# FASCICULE DE BREVET EUROPEEN

(11) **EP 0 629 634 B1**
(45) Date de publication et mention de la délivrance du brevet: **25.02.1998**
(21) Numéro de dépôt: 94401103.0
(22) Date de dépôt: 18.05.1994
(51) Int. Cl.: C07J 41/00, C07J 9/00

(54) **Procédé pour la préparation d'acides taurocholaniques**
Verfahren zur Herstellung von Taurocholansäure
Process for the preparation of taurocholic acids

(30) Priorité: 20.05.1993 IT TO930346
(43) Date de publication de la demande: 21.12.1994
(73) Titulaire: SANOFI, 75008 Paris (FR)
(72) Inventeur: Arosio, Roberto, I-22040 Civate (Como) (IT); Rossetti, Vittorio, I-20100 Milano (IT)
(74) Mandataire: Gillard, Marie-Louise

(56) Documents cités:
- EP-A- 0 135 782
- EP-A- 0 400 695
- DE-A- 3 736 918
- US-A- 4 088 760
- J. FALBE 'METHODEN DER ORGANISCHEN CHEMIE, ERWEITERUNG- UND FOLGEBÄNDE ZUR VIERTEN AUFLAGE, BAND E5, CARBONSÄUREN UND CARBONSÄURE-DERIVATE' 1985 , GEORG THIEME VERLAG , STUTTGART, DE * page 635 - page 636 * * page 978 - page 981 *
- Chemical Rubber Co Handbook of Tables for Organic Compound Identification, 3rd Ed. pp 319
- Règles de Nomenclature pour la Chimie Organique (Règles 1965) pp 239

## Description

La présente invention concerne un procédé pour la préparation de dérivés d'acides cholaniques conjugués avec la taurine, ci-après désignés acides taurocholaniques de formule (I) 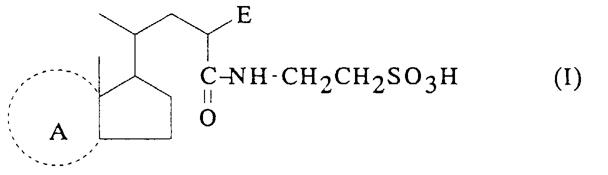 dans laquelle A complète la structure 5β-cyclopentaneperhydrophénanthrénique d'un acide cholanique et E représente l'hydrogène ou un groupe méthyle, ainsi que de leurs sels pharmaceutiquement acceptables.

Selon la présente invention, le terme "acides cholaniques" inclut l'acide cholanique lui-même (ou acide 5β-cholan-24-oïque) non substitué ou substitué, ainsi que les produits découlant de son oxydation au niveau de la structure cyclopentaneperhydrophénanthrénique, comme, par exemple, les dérivés dihydroxy et trihydroxy sur ladite structure, notamment l'acide ursodésoxycholique (3α,7β-dihydroxy), l'acide chénodésoxycholique (3α,7α-dihydroxy), l'acide hyodésoxycholique (3α,6α-dihydroxy), l'acide désoxycholique (3α-hydroxy), l'acide cholique (3α,7α,12α-trihydroxy), l'acide ursocholique (3α,7β,12α-trihydroxy), l'acide hyocholique (3α,6α,12α-trihydroxy).

Les acides taurocholiques, notamment l'acide tauroursodésoxycholique et l'acide taurochénodésoxycholique, sont des composés présents dans l'organisme, qui sont stockés dans la vésicule biliaire et qui, secrétés avec la bile, participent à l'absorption intestinale des lipides.

L'utilisation de l'acide ursodésoxycholique dans la thérapie trouve son application dans le traitement de différentes altérations de la fonction biliaire notamment dans les dysfonctionnements dûs à la bile sur-saturée de cholestérol pour la dissolution des calculs dans la vésicule biliaire ou pour en empêcher la formation.

Certaines synthèses chimiques des acides taurocholaniques, plus particulièrement de l'acide tauroursodésoxycholique (TUDCA) et de l'acide 23-méthyl-tauroursodésoxycholique (23-TMUDCA), ont été décrites en littérature.

Le brevet italien 1167038, par exemple, décrit la préparation de l'acide tauroursodésoxycholique par condensation directe de l'acide ursodésoxycholique et de la taurine en présence d'un agent de condensation.

Le brevet italien 1197331 décrit la préparation de l'acide tauroursodésoxycholique qui utilise l'azide de l'acide ursodésoxycholique comme intermédiaire de réaction.

Le brevet EP135782 décrit des dérivés de l'acide 23-méthylcholanique et plus particulièrement la préparation du 23-TMUDCA.

La synthèse d'acides taurocholaniques qui, actuellement, semble être la plus utilisée est celle illustrée dans le brevet italien 1212092 qui décrit la préparation d'amides de l'acide ursodésoxycholique, dont celle de l'acide tauroursodésoxycholique, par l'intermédiaire d'un anhydride mixte préparé par réaction de l'acide ursodésoxycholique avec un chloroformiate d'alkyle ou de phényle et par traitement dudit anhydride mixte avec la taurine. Cette réaction est particulièrement délicate parce que l'anhydride qui se forme est un composé instable qui se décompose facilement; de plus, les chloroformiates utilisés sont des composés très volatiles bien connus comme puissants réactifs chimiques notoirement toxiques. Il est donc nécessaire que les conditions de réaction, notamment la température, soient soigneusement contrôlées. De toute façon, même en opérant avec précaution, les rendements ne sont pas très satisfaisants.

Il a été maintenant trouvé qu'en faisant réagir un dérivé de l'acide cholanique avec un chlorure d'acide convenable, on obtient des anhydrides mixtes particulièrement adaptés pour la réaction suivante avec la taurine.

Ainsi, selon un de ses aspects, la présente invention concerne un procédé pour la préparation d'un acide taurocholanique de formule (I) 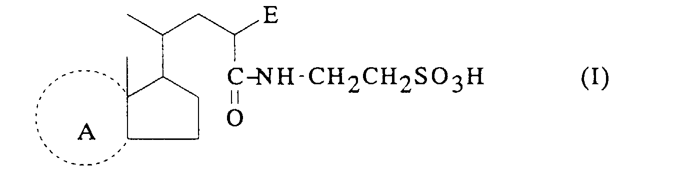 dans laquelle A et E sont tels que définis ci-dessus, ou d'un de ses sels, caractérisé en ce que
(a) on traite un acide cholanique de formule (II) 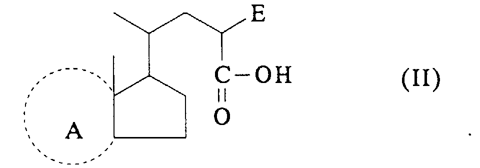 dans laquelle A et E sont tels que définis ci-dessus, avec un chlorure d'acide de formule (III) 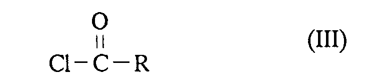 dans laquelle R est le résidu d'un hydrocarbure lié au groupe carbonyle par un carbone tertiaire, en présence d'une amine tertiaire, éventuellement dans un solvant organique aprotique polaire miscible ou partiellement miscible à l'eau ; puis
(b) on traite l'anhydride mixte intermédiaire ainsi obtenu de formule (IV) 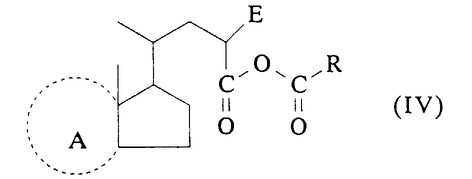 dans laquelle A, E et R sont tels que définis ci-dessus, avec la taurine et on isole le produit de formule (I) obtenu tel quel ou sous forme d'un de ses sels.
   Si le produit final est isolé sous forme de sel, celui-ci peut être pharmaceutiquement acceptable ou non. Dans ce dernier cas, l'acide taurocholanique est isolé par neutralisation dudit sel et éventuellement transformé en un autre sel pharmaceutiquement acceptable de préférence un sel alcalin ou alcalino-terreux. Selon un aspect préféré, le procédé de la présente invention conduit à l'acide tauroursodésoxycholique, à l'acide 23-méthyl-tauroursodésoxycholique, à l'acide taurochénodésoxycholique et à leurs sels pharmaceutiquement acceptables.
   Dans l'étape (a) du procédé, le chlorure d'acide de formule (III) est dérivé d'un acide carboxylique de formule (III')

   R-COOH (III')

   dans laquelle R est le résidu d'un hydrocarbure lié au carbonyle par un carbone tertiaire, contenant de préférence de 3 à 24 atomes de carbone. Par "hydrocarbure" on désigne notamment les hydrocarbures aliphatiques ou cycloaliphatiques saturés ou insaturés et les hydrocarbures aromatiques. Plus particulièrement R peut être un groupe alkyle ou alkényle tertiaire tel que 2-propényle, *tert*-butyle, 2-méthylbutyle (*tert*-pentyle), triéthylméthyle; un groupe aromatique, tel qu'un phényle substitué ou non substitué, par exemple phényle, p-méthylphényle, un naphtyle, un anthracényle, un phénanthrényle, un 5,6,7,8-tétrahydronapht-1-yle ou un 5,6,7,8-tétrahydronapht-2-yle ; un groupe cycloaliphatique, tel que 1-cyclohexényle ou 1-méthylcyclohexyle; de préférence R représente *tert*-butyle ou phényle.

Les chlorures d'acide de formule (III) qui sont stériquement encombrés donnent des anhydrides mixtes qui favorisent de façon presque sélective le rattachement de la taurine au groupe carbonyle de l'acide cholanique, en conduisant à une forte augmentation du rendement final.

Les chlorures de formule (III) qui peuvent être utilisés sont par exemple le chlorure de pivaloyle, le chlorure de benzoyle, le chlorure de 2,2-diméthylvaléroyle, le chlorure de 2-naphtoyle, le chlorure de 1-naphtoyle, le chlorure de l'acide 1-méthylcyclohexanecarboxylique méthylcyclohexanecarboxylique ou le chlorure de l'acide le chlorure de pivaloyle et de benzoyle s'étant montrés particulièrement convenables.

En général, on utilise une quantité équimolaire ou, de préférence, un petit excès de chlorure d'acide de formule (III) par rapport à l'acide cholanique de formule (II).

La condensation de l'étape (a) est effectuée en présence d'une amine tertiaire, de préférence choisie parmi celles utilisées couramment comme bases de Lewis, par exemple la triéthylamine, la tributylamine, les N-alkylpipéridines, notamment la N-méthylpipéridine, la pyridine ou la 4-diméthylaminopyridine.

Généralement, on utilise une quantité équimolaire d'une telle amine par rapport à l'acide de départ mais il est possible d'employer un excès d'amine, par exemple lorsqu'on souhaite effectuer la réaction sans utiliser d'autres solvants.

De préférence, la réaction est réalisée en présence d'un solvant organique aprotique, polaire ou apolaire, miscible ou partiellement miscible à l'eau, qui n'interfère pas avec le déroulement de la réaction. De tels solvants sont par exemple le dioxane, l'acétone, la pyridine, seuls ou en mélange entre eux.

La température ne constitue pas un facteur critique dans la conduction de la réaction, la dite température pouvant être comprise entre 0°C et 50°C, de préférence entre 10°C et la température ambiante.

La réaction de l'étape (a) s'amorce immédiatement et s'achève dans un temps court, en quelques heures au maximum. Lorsqu'on le souhaite, on peut aussi maintenir le mélange réactionnel sous agitation plus longtemps car l'anhydride de formule (IV) qui se forme est stable.

A la fin de l'étape (a), on filtre les sels éventuels qui sont précipités et, éventuellement, on isole l'anhydride mixte. On passe ensuite à l'étape (b) qui consiste en la réaction avec la taurine soit par addition directe de celle-ci au mélange réactionnel soit par addition à une solution de l'anhydride mixte préalablement isolé dans un solvant convenable.

Dans l'étape (b), la taurine est utilisée sous la forme d'une solution aqueuse basique mais lorsque la taurine est ajoutée au mélange réactionnel obtenu à la fin de l'étape (a) dans laquelle un fort excès d'amines tertiaires a été utilisé, la taurine telle quelle peut être tout simplement ajoutée audit mélange réactionnel.

Il est nécessaire d'utiliser une quantité au moins équimolaire de taurine par rapport à l'acide cholanique de formule (II) mais, pour assurer un meilleur rendement de réaction, il est préférable d'utiliser un excès de taurine, avantageusement de 30 à 40%.

L'addition de taurine est effectuée de préférence en refroidissant légèrement le mélange réactionnel. Une fois que l'addition est terminée, on laisse remonter graduellement la température en la maintenant de préférence entre 10°C et la température ambiante. En général, la réaction s'achève en quelques heures, compte tenu des différents paramètres de réaction, mais son déroulement peut être de toute façon vérifié par les techniques chromatographiques usuelles.

A la fin de la réaction, l'excès éventuel de taurine qui n'a pas réagi est filtré et éliminé. L'acide taurocholanique de formule (I) ainsi obtenu est séparé et purifié selon les méthodes classiques. Généralement, il suffit d'ajouter au mélange réactionnel un solvant qui permet la formation d'un azéotrope avec l'eau pour que le produit de réaction précipite; plus rarement, il est nécessaire d'éliminer d'abord l'eau par distillation azéotropique afin d'obtenir la précipitation.

Alternativement, une fois la réaction terminée et l'excès de taurine éliminé par filtration, on peut utiliser une résine échangeuse d'ions pour une meilleure purification du produit. Dans ce cas, on fait passer la solution sur deux résines: d'abord sur une résine fortement acide, puis sur une résine basique et, seulement après cette double opération, on ajoute le solvant qui permet la séparation du produit final.

Si on le souhaite, le produit obtenu peut être ultérieurement purifié par cristallisation dans des solvants convenables.

Les anhydrides mixtes de formule (IV) 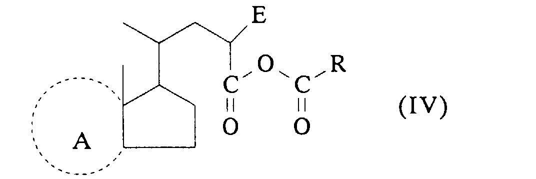 dans laquelle A, E et R sont tels que définis ci-dessus, sont des composés nouveaux et représentent un autre aspect de la présente invention. Les anhydrides de formule (IV), dans laquelle A complète la structure de l'acide ursodésoxycholique ou de l'acide chénodésoxycholique et E et R sont tels que définis ci-dessus, sont des composés particulièrement avantageux, les anhydrides mixtes de l'acide ursodésoxycholique, de l'acide 23-méthyl-ursodésoxycholique et de l'acide chénodésoxycholique avec l'acide triméthylacétique ou l'acide benzoïque sont particulièrement préférés.

Lorsque l'on veut isoler les anhydrides de formule (IV), les sels précipités à la fin de ladite étape (a) sont éliminés par filtration et la solution est évaporée sous vide à une température non supérieure à 30°C pour obtenir les composés (IV).

Les exemples suivants illustrent l'invention sans cependant la limiter.

### EXEMPLE 1

### Acide tauroursodésoxycholique

### Préparation

A une suspension de 9,6 g (24,45 mmole) d'acide ursodésoxycholique dans 60 ml de dioxane, on ajoute goutte à goutte, à la température de 20°C, 2,5 g (24,75 mmole) de triéthylamine. Une fois solubilisé, on refroidit le mélange jusqu'à 10°C et on y ajoute lentement 2,98 g (24,70 mmole) de chlorure de pivaloyle puis on laisse spontanément remonter la température à 20°C. On filtre les sels formés et on lave le précipité avec du dioxane, qu'on ajoute à la solution. On ajoute à la solution obtenue 3,84 g (30,72 mmole) de taurine dissoute dans 24 ml de NaOH 1N et on agite pendant 3 heures à 15°C. On peut suivre la réaction par chromatographie sur couche mince en éluant avec CHCl₃/MeOH/AcOH=70/30/5.

### Purification

- 1^{ère} METHODE
   On filtre pour éliminer la taurine qui n'a pas réagi; on refroidit la solution jusqu'à 0°-5°C et on y ajoute lentement 600 ml d'acétone. On obtient la séparation d'une masse gluante et on élimine le solvant par décantation. On dissout le résidu dans 30 ml d'eau, on y ajoute 100 ml de toluène et on distille l'azéotrope pour éliminer l'eau. On obtient un solide qu'on filtre aisément et on le lave au toluène.
- 2^{ème} METHODE
   On filtre la taurine qui n'a pas réagi et on fait passer la solution sur une colonne de 20 ml d'une résine cationique Rélite CF (forme acide) et ensuite sur une résine Rélite GH1 (base libre) lentement. On refroidit la solution à 0°C et on y ajoute 600 ml d'acétone, on filtre le précipité formé et on le lave à l'acétone.
   P.f. 135-141°C; I.R. (nujol) cm⁻¹: 1050 (-SO₃); 1545 et 1650 (amide); 1200 (-SO₃).

### EXEMPLE 2

### Acide tauroursodésoxycholique

On suit la méthode de l'exemple 1 en utilisant 3,48 g (24,70 mmole) de chlorure de benzoyle au lieu de 2,98 g de chlorure de pivaloyle.

### EXEMPLE 3

### Acide taurochénodésoxycholique

### Préparation

On suit la méthode de l'exemple 1 en utilisant 9,6 g d'acide chénodésoxycholique au lieu de l'acide ursodésoxycholique.

### Purification

On élimine par filtration l'excès de taurine qui n'a pas réagi, on refroidit jusqu'à 0°C et on ajoute lentement 600 ml d'acétone à la solution. On filtre le solide blanc qui se sépare et on le lave à l'acétone.
I.R. (nujol) cm⁻¹: 1050 (-SO₃); 1545 et 1650 (amide); 1200 (-SO₃).

### EXEMPLE 4

### Acide taurochénodésoxycholique

On suit la méthode de l'exemple 3 en utilisant 3,48 g (24,70 mmole) de chlorure de benzoyle au lieu du chlorure de pivaloyle.
Les caractéristiques des produits obtenus par les réactions des exemples 1 à 4 sont illustrées dans le tableau I ci-dessous.

**TABLEAU I**

| Exemple | Rendement | taurine résiduelle | acide de départ résiduel |
|---|---|---|---|
| 1 | 77,4 % | 1 % | 1 % |
| 2^{ème} méthode de purification | | | |
| 2 | 73,9 % | 1 % | 1 % |
| 2^{ème} méthode de purification | | | |
| 3 | 75,4 % | 1 % | 2 % |
| 4 | 60 % | 0,5 % | 0,5 % |

### EXEMPLE 5

### Anhydride pivalique de l'acide ursodésoxycholique

On suit la méthode de l'exemple 1 jusqu'à la filtration des sels. On évapore le solvant sous pression réduite à une température non supérieure à 30°C. On dissout le résidu dans du chloroforme et on extrait à l'eau. On sépare les deux phases, on sèche la phase organique sur du sulfate de sodium et on évapore le solvant sous pression réduite à basse température. On obtient ainsi l'anhydride 3α,7β-dihydroxy-5β-cholan-24-oïque-triméthylacétique.
I.R. (nujol) cm⁻¹: 1810, 1720.

Les données plus significatives du spectre ¹³C-RMN sont consignées dans le tableau suivant

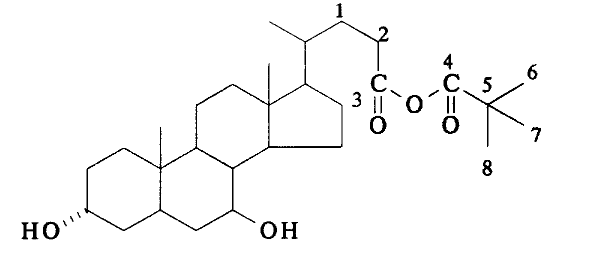

| ATOME N° | PPM |
|---|---|
| 1 | 28,52 |
| 2 | 29,88 |
| 3 | 168,78 |
| 4 | 173,37 |
| 5 | 35,05 |
| 6 | 24,92 |
| 7 | 24,92 |
| 8 | 24,92 |

### EXEMPLE 6

### Anhydride benzoïque de l'acide ursodésoxycholique

On suit la méthode de l'exemple 5 en utilisant 3,48 g (24,70 mmole) de chlorure de benzoyle au lieu du chlorure de pivaloyle. On obtient ainsi l'anhydride 3α,7β-dihydroxy-5β-cholan-24-oïque-benzoïque.
Les données plus significatives du spectre ¹³C-RMN sont consignées dans le tableau suivant

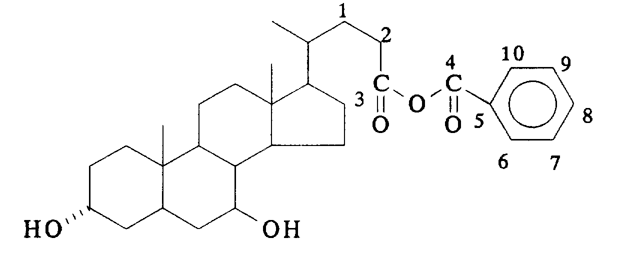

| ATOME N° | PPM |
|---|---|
| 1 | 28,52 |
| 2 | 29,88 |
| 3 | 168,76 |
| 4 | 161,66 |
| 5 | 129,39 |
| 6 | 130,57 |
| 7 | 128,84 |
| 8 | 131,89 |
| 9 | 128,84 |
| 10 | 130,57 |

## Revendications

1. Procédé pour la préparation d'un dérivé de l'acide taurocholanique de formule (I) 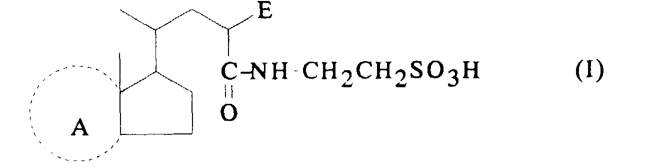 dans laquelle A complète la structure 5β-cyclopentaneperhydrophénanthrénique d'un acide cholanique et E représente l'hydrogène ou un groupe méthyle, ou d'un de ses sels, caractérisé en ce que
(a) on traite un acide cholanique de formule (Il) 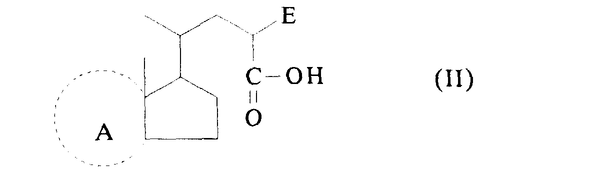 dans laquelle A et E sont tels que définis ci-dessus, avec un chlorure d'acide de formule (III) 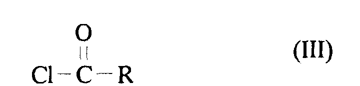 dans laquelle R est le résidu d'un hydrocarbure lié au groupe carbonyle par un carbone tertiaire, en présence d'une amine tertiaire, éventuellement dans un solvant organique aprotique polaire miscible ou partiellement miscible à l'eau puis
(b) on traite l'anhydride mixte intermédiaire ainsi obtenu de formule (IV) 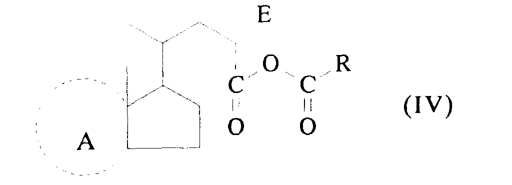 dans laquelle A, E et R sont tels que définis ci-dessus, avec une quantité au moins équimolaire de taurine par rapport à l'acide cholanique de formule (Il) et on isole le produit de formule (I) obtenu tel quel ou sous forme d'un de ses sels.

2. Procédé selon la revendication 1, caractérisé en ce que A complète la structure de l'acide ursodésoxycholique.

3. Procédé selon la revendication 1, caractérisé en ce que A complète la structure de l'acide chénodésoxycholique.

4. Procédé selon l'une quelconque des revendications 1 à 3, caractérisé en ce que R est le résidu d'un hydrocarbure lié au carbonyle par un carbone tertiaire contenant de 3 à 24 atomes de carbone.

5. Procédé selon la revendication 4, caractérisé en ce que le composé de formule (III) est choisi parmi le chlorure de pivaloyle, le chlorure de benzoyle, le chlorure de 2,2-diméthylvaléroyle, le chlorure de 2-naphtoyle, le chlorure de 1-naphtoyle, le chlorure de l'acide 1-méthylcyclohexanecarboxylique ou le chlorure de l'acide 1-cyclohexènecarboxylique.

6. Procédé selon la revendication 5, caractérisé en ce que le composé de formule (III) est choisi parmi le chlorure de pivaloyle ou le chlorure de benzoyle.

7. Procédé selon l'une quelconque des revendications 1 à 6, caractérisé en ce que l'amine tertiaire est choisie parmi la triéthylamine, la tributylamine, la N-méthylpipéridine, la pyridine ou la 4-diméthylaminopyridine.

8. Procédé selon l'une quelconque des revendications 1 à 7, caractérisé en ce que le composé de formule (III) est utilisé en quantité équimolaire ou en faible excès par rapport à l'acide cholanique de formule (II).

9. Procédé selon l'une quelconque des revendications 1 à 8, caractérisé en ce que la réaction est réalisée dans un solvant organique aprotique, polaire ou apolaire, miscible ou partiellement miscible à l'eau.

10. Procédé selon l'une quelconque des revendications 1 à 9, caractérisé en ce que la taurine est ajoutée au mélange réactionnel dans une solution aqueuse basique.

11. Procédé selon l'une quelconque des revendications 1 à 10, caractérisé en ce que la taurine est utilisée en excès par rapport à l'acide cholanique de formule (II).

12. Anhydrides de formule (IV) 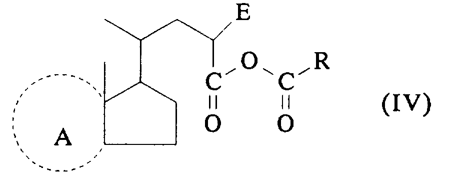 dans laquelle A complète la structure 5β-cyclopentaneperhydrophénanthrénique d'un acide cholanique, E représente l'hydrogène ou un groupe méthyle et R est le résidu d'un hydrocarbure lié au groupe carbonyle par un carbone tertiaire.

13. Anhydride selon la revendication 12, caractérisé en ce que R est le résidu d'un hydrocarbure lié au carbonyle par un carbone tertiaire contenant de 3 à 24 atomes de carbone.

14. Anhydride selon la revendication 12, caractérisé en ce que A complète la structure de l'acide ursodésoxycholique et R représente un groupe tert-butyle ou phényle.

15. Anhydride selon la revendication 12, caractérisé en ce que A complète la structure de l'acide chénodésoxycholique et R représente un groupe tert-butyle ou phényle.

16. Anhydride 3α,7β-dihydroxy-5β-cholan-24-oïque-benzoïque.

17. Anhydride 3α,7β-dihydroxy-5β-cholan-24-oïque-triméthylacétique.

## Patentansprüche

1. Verfahren zur Herstellung von Taurocholansäurederivaten der Formel (I) 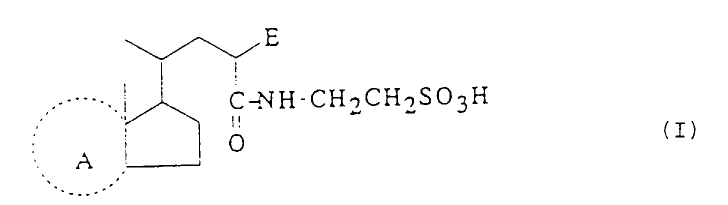 worin A die 5β-Cyclopentanperhydrophenanthrenstruktur einer Cholansäure vervollständigt und E Wasserstoff oder eine Methylgruppe bedeutet,
oder eines Salzes dieser Derivate,
gekennzeichnet durch folgende Schritte:
(a) Umsetzung einer Cholansäure der Formel (II) 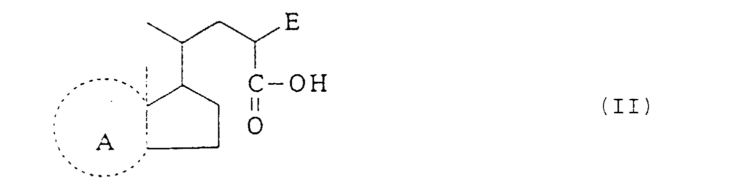 worin A und E die oben angegebenen Bedeutungen aufweisen,
mit einem Säurechlorid der Formel (III) 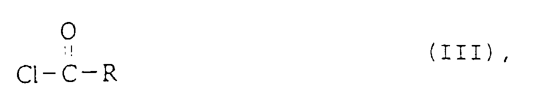 worin R den Rest eines Kohlenwasserstoffs bedeutet, der an die Carbonylgruppe über ein tertiäres Kohlenstoffatom gebunden ist,
in Gegenwart eines tertiären Amins gegebenenfalls in einem organischen aprotischen polaren mit Wasser mischbaren oder teilweise mischbaren Lösungsmittel, und
(b) Umsetzung des so als Zwischenprodukt hergestellten gemischten Anhydrids der Formel (IV) 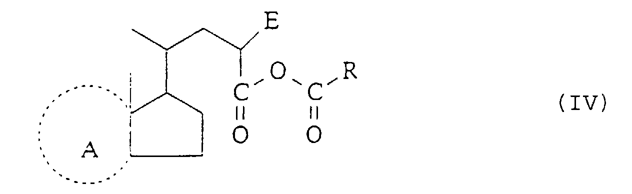 worin A, E und R die oben angegebenen Bedeutungen aufweisen,
mit einer zumindest äquimolaren Menge Taurin, bezogen auf die Cholansäure der Formel (II), und Abtrennung des erhaltenen Produkts der Formel (I) in der Form, in der es hergestellt wurde, oder in Form eines seiner Salze.

2. Verfahren nach Anspruch 1, dadurch gekennzeichnet, daß A die Struktur der Ursodesoxycholsäure vervollständigt.

3. Verfahren nach Anspruch 1, dadurch gekennzeichnet, daß A die Struktur der Chenodesoxycholsäure vervollständigt.

4. Verfahren nach einem der Ansprüche 1 bis 3, dadurch gekennzeichnet, daß R der Rest eines Kohlenwasserstoffs ist, der an das Carbonyl über ein tertiäres Kohlenstoffatom gebunden ist und 3 bis 24 Kohlenstoffatome aufweist.

5. Verfahren nach Anspruch 4, dadurch gekennzeichnet, daß die Verbindung der Formel (III) unter Pivaloylchlorid, Benzoylchlorid, 2,2-Dimethylvaleroylchlorid, 2-Naphthoylchlorid, 1-Naphthoylchlorid, 1-Methylcyclohexancarbonsäurechlorid oder 1-Cyclohexencarbonsäurechlorid ausgewählt ist.

6. Verfahren nach Anspruch 5, dadurch gekennzeichnet, daß die Verbindung der Formel (III) unter Pivaloylchlorid oder Benzoylchlorid ausgewählt ist.

7. Verfahren nach einem der Ansprüche 1 bis 6, dadurch gekennzeichnet, daß das tertiäre Amin unter Triethylamin, Tributylamin, N-Methylpiperidin, Pyridin oder 4-Dimethylaminopyridin ausgewählt ist.

8. Verfahren nach einem der Ansprüche 1 bis 7, dadurch gekennzeichnet, daß die Verbindung der Formel (III) in einem äquimolaren Anteil oder in geringem Überschuß, bezogen auf die Cholansäure der Formel (II), verwendet wird.

9. Verfahren nach einem der Ansprüche 1 bis 8, dadurch gekennzeichnet, daß die Umsetzung in einem polaren oder apolaren aprotischen mit Wasser mischbaren oder teilweise mischbaren organischen Lösungsmittel durchgeführt wird.

10. Verfahren nach einem der Ansprüche 1 bis 9, dadurch gekennzeichnet, daß das Taurin dem Reaktionsgemisch in einer wäßrigen basischen Lösung zugegeben wird.

11. Verfahren nach einem der Ansprüche 1 bis 10, dadurch gekennzeichnet, daß das Taurin, bezogen auf die Cholansäure der Formel (II), im Überschuß verwendet wird.

12. Anhydride der Formel (IV) 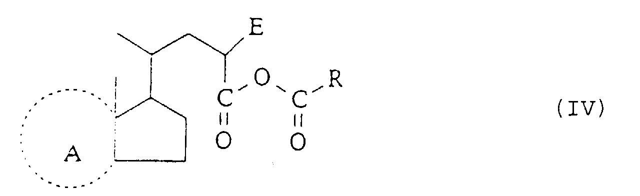 worin A die 5β-Cyclopentanperhydrophenanthrenstruktur einer Cholansäure vervollständigt, E Wasserstoff oder eine Methylgruppe bedeutet und R der Rest eines Kohlenwasserstoffs ist, der an die Carbonylgruppe über ein tertiäres Kohlenstoffatom gebunden ist.

13. Anhydrid nach Anspruch 12, dadurch gekennzeichnet, daß R der Rest eines Kohlenwasserstoffs ist, der über ein tertiäres Kohlenstoffatom an das Carbonyl gebunden ist und 3 bis 24 Kohlenstoffatome aufweist.

14. Anhydrid nach Anspruch 12, dadurch gekennzeichnet, daß A die Struktur der Ursodesoxycholsäure vervollständigt und R eine tert.-Butylgruppe oder Phenylgruppe bedeutet.

15. Anhydrid nach Anspruch 12, dadurch gekennzeichnet, daß A die Struktur der Chenodesoxycholsäure vervollständigt und R eine tert.-Butylgruppe oder Phenylgruppe bedeutet.

16. 3α,7β-Dihydroxy-5β-cholan-24-säure-Benzoesäureanhydrid.

17. 3α,7β-Dihydroxy-5β-cholan-24-säure-trimethylessigsäureanhydrid.

## Claims

1. A process for the preparation of a taurocholanic acid derivative of formula (I): 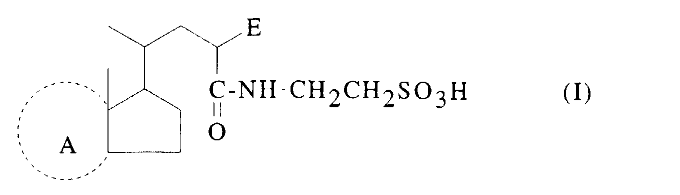 in which A completes the 5β-cyclopentaneperhydrophenanthrene structure of a cholanic acid and E is hydrogen or a methyl group, or one of its salts, characterised in that
(a) a cholanic acid of formula (II): 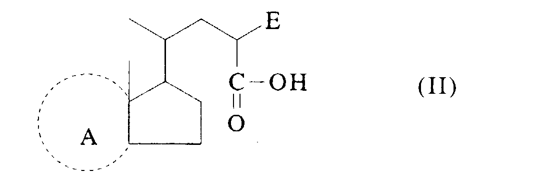 in which A and E are as defined above, is treated with an acid chloride of formula (III): 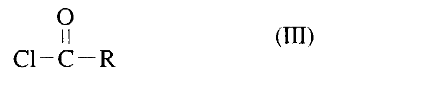 in which R is a hydrocarbon residue bonded to the carbonyl group by a tertiary carbon, in the presence of a tertiary amine, optionally in a polar aprotic organic solvent which is miscible or partially miscible with water; and then
(b) the thus obtained intermediate mixed anhydride of formula (IV): 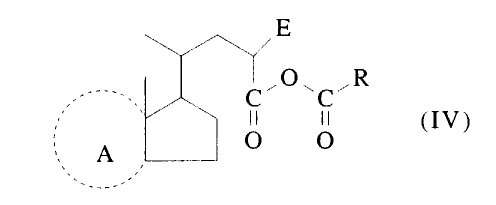 in which A, E and R are as defined above, is treated with at least an equimolar amount of taurine relative to the cholanic acid of formula (II) and the obtained product of formula (I) is isolated as such or in the form of one of its salts.

2. A process according to claim 1, characterised in that A completes the structure of ursodeoxycholic acid.

3. A process according to claim 1, chatacterised in that A completes the structure of chenodeoxycholic acid.

4. A process according to any one of claims 1 to 3, characterised in that R is a hydrocarbon residue bonded to the carbonyl by a tertiary carbon and containing from 3 to 24 carbon atoms.

5. A process according to claim 4, characterised in that the compound of formula (III) is selected from pivaloyl chloride, benzoyl chloride, 2,2-dimethylvaleroyl chloride, 2-naphthoyl chloride, 1-naphthoyl chloride, 1-methylcyclohexanecarboxylic acid chloride and cyclohex-1-enecarboxylic acid chloride.

6. A process according to claim 5, characterised in that the compound of formula (III) is selected from pivaloyl chloride and benzoyl chloride.

7. A process according to any one of claims 1 to 6, characterised in that the tertiary amine is selected from triethylamine, tributylamine, N-methylpiperidine, pyridine and 4-dimethylaminopyridine.

8. A process according to any one of claims 1 to 7, characterised in that the compound of formula (III) is used in an equimolar amount or in slight excess relative to the cholanic acid of formula (II).

9. A process according to any one of claims 1 to 8, characterised in that the reaction is carried out in a polar or apolar, aprotic organic solvent which is miscible or partially miscible with water.

10. A process according to any one of claims 1 to 9, characterised in that the taurine is added to the reaction mixture in a basic aqueous solution.

11. A process according to any one of claims 1 to 10, characterised in that the taurine is used in an excess relative to the cholanic acid of formula (II).

12. Anhydrides of formula (IV): 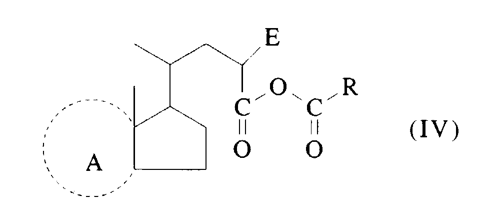 in which A completes the 5β-cyclopentaneperhydrophenanthrene structure of a cholanic acid, E is hydrogen or a methyl and R is a hydrocarbon residue bonded to the carbonyl group by a tertiary carbon.

13. An anhydride according to claim 12, characterised in that R is a hydrocarbon residue bonded to the carbonyl by a tertiary carbon and containing from 3 to 24 carbon atoms.

14. An anhydride according to claim 12, characterised in that A completes the structure of ursodeoxycholic acid and R is a tert-butyl or phenyl group.

15. An anhydride according to claim 12, characterised in that A completes the structure of chenodeoxycholic acid and R is a tert-butyl or phenyl group.

16. The benzoic anhydride of 3α,7β-dihydroxy-5β-cholan-24-oic acid.

17. The trimethylacetic anhydride of 3α,7β-dihydroxy-5β-cholan-24-oic acid.
